# EUROPEAN PATENT SPECIFICATION

(11) **EP 1 985 330 B1**
(45) Date of publication and mention of the grant of the patent: **02.06.2010**
(21) Application number: 08251489.4
(22) Date of filing: 22.04.2008
(51) Int. Cl.: A61M 25/01

(54) **Catheter control**
Kathetersteuerung
Contrôle de cathéter

(30) Priority: 23.04.2007 US 925992 P
(43) Date of publication of application: 29.10.2008
(73) Proprietor: Cathrx Ltd, Homebush Bay, NSW 2127 (AU)
(72) Inventor: Anderson, Neil Lawrence, Roseville, New South Wales 2069 (AU); Ogle, David, Cowan, New South Wales 2081 (AU); Smith, Matthew Lee, Bondi, New South Wales 2026 (AU)
(74) Representative: Nettleton, John Victor

(56) References cited:
- WO-A-00/07500
- WO-A-2007/008967
- US-A- 4 753 248
- US-A1- 2002 177 789

## Description

### Field

This invention relates, generally, to catheter control and more particularly, to a catheter control assembly. Preferably, but not essentially, the catheter control assembly is remotely controlled.

### Background

Generally, in the manipulation of catheters in cardiovascular procedures, the catheter sheath is inserted, by an introducer, through the vasculature of a patient until the distal end of the catheter is at the desired location in the patient's heart. It will be appreciated that to manipulate the catheter through the patient's vasculature requires great dexterity and steadiness on the part of the clinician. The more stable the catheter, the smaller the incision for minimally invasive surgery can be with the resultant improved convalescence time and costs associated with such convalescence.

In addition, use is generally made of radio opaque markers on the catheters for determining the location of the catheter within the patient's vasculature. These radio opaque markers are detected by X-ray fluoroscopy which means that, when the clinician is conducting a procedure in proximity to the patient, the clinician is exposed to radiation arising due to the X-ray fluoroscopy. It would be beneficial to be able to remove the clinician from exposure to this radiation. Thus, to be able to control manipulation of the catheter remotely would be advantageous.

US 4,753,248 to Engler et al describes an automated temperature scanning system for monitoring hyperthermia treatment. The system has a linear drive assembly operatively connected to a non-rotational screw shaft which is linearly translated by a stepper motor. A first tube having a thermometric probe concentrically positioned therein is fixedly secured to the linear drive assembly so as to be motivated thereby. A second tube is secured at one end to an interstitial catheter and is adapted to be at least partially slidably received within the first tube so that linear movement of the first tube toward the second tube slidably moves the thermometric probe within the second tube and an interstitial catheter associated therewith. A computer is electrically connected to the stepper motor to control the movement of the thermometric probe utilising real time data generated thereby.

### Summary

According to a first aspect of the invention, there is provided a catheter control assembly which includes
a support structure;
a drive arrangement supported by the support structure;
an elongate displacement mechanism associated with the drive arrangement to be displaced at least axially under the action of the drive arrangement; and
a carrier, for mounting at least a part of a catheter handle, mounted on the displacement mechanism, the carrier comprising a plurality of independently displaceable receiving formations, each receiving formation, in use, receiving a part of the catheter handle and at least two of the receiving formations being axially displaceable relative to each other **characterised in that** the displacement mechanism includes a telescopic arm assembly extending from the support structure and on which the carrier is mounted, the telescopic arm assembly comprising a plurality of telescopically arranged arms and each arm mounting one of the receiving formations of the carrier.

The telescopic arm assembly may be at least axially and rotatably arranged relative to the support structure for effecting axial and rotational displacement of the carrier relative to the support structure. The telescopic arm assembly may include at least a first arm and a second arm, the second arm of the telescopic arm assembly displacing one receiving formation of the carrier relative to at least one other receiving formation of the carrier. The telescopic arm assembly may include at least one further arm for displacing another receiving formation of the carrier relative to at least one other receiving formation of the carrier.

Preferably, the carrier is removably attached to the telescopic arm assembly.

The assembly may include a controller for controlling operation of the drive arrangement to effect displacement of the displacement mechanism. Preferably, the controller is arranged remotely of the support structure. In an embodiment, the controller may be shaped to mimic the appearance and feel of the catheter handle.

The drive arrangement may comprise a plurality of drive motors, each drive motor driving a part of the displacement mechanism. Each drive motor may be a stepper motor. It will be appreciated that each arm of the telescopic arm assembly of the displacement mechanism may have a drive motor associated with it, rotary motion of the drive motor being translated into linear motion of the relevant telescopic arm assembly. The drive arrangement may further include a rotation control motor for effecting rotary control of the displacement mechanism. The rotation control motor may also be a stepper motor.

The support structure may include a housing in which the drive arrangement is received with the displacement mechanism extending from the housing.

In an embodiment, the carrier may be in the form of a cradle in which at least a part of the catheter handle is receivable. The cradle may include an elongate body member containing the receiving formations and a mounting arrangement arranged at an opposed end of the body member for mounting the body member to a displacement mechanism of a catheter control assembly.

The mounting arrangement may comprise a plurality of independently displaceable mounting members and each receiving formation may be connected to an associated mounting member via a pair of transversely spaced limbs. The limbs on each side of the body member may be shaped to nest within each other.

In another embodiment, the carrier may comprise a plurality of discrete components mounted on one of the arms of the telescopic arm assembly, each component defining a mounting formation for receiving a part of the catheter handle. In this embodiment, each arm may comprise a pair of transversely spaced members.

According to a second aspect of the invention, there is provided a catheter control assembly which includes
a carrier for mounting a catheter handle, the carrier comprising a plurality of independently displaceable receiving formations, each receiving formation, in use, receiving a part of the catheter handle;
a telescopic arm assembly on which the carrier is mounted, the telescopic arm assembly comprising a plurality of telescopically arranged arms and each arm mounting a receiving formation; and
a drive arrangement which acts on the arms of the telescopic arm assembly for effecting displacement of the telescopic arm assembly and the carrier and at least certain arms of the telescopic arm assembly and the receiving formations of the carrier.

The assembly may include a controller arranged remotely of the carrier for controlling operation of the drive arrangement.

According to a third aspect of the invention, there is provided a remote control unit for remotely controlling a catheter, the remote control unit including
a drive arrangement which acts on the carrier for effecting displacement of the carrier and the receiving formations of the carrier;
an elongate displacement mechanism associated with the drive arrangement to be displaced at least axially under the action of the drive arrangement;
a carrier for mounting at least a part of a catheter handle mounted on the displacement mechanism, the carrier comprising a plurality of independently displaceable receiving formations, each receiving formation, in use, receiving a part of the catheter handle and at least two of the receiving formations being axially displaceable relative to each other; and
a controller arranged remotely of the drive arrangement for controlling operation of the drive arrangement.

The displacement mechanism may include a telescopic arm assembly extending from the support structure and on which the carrier is mounted, the telescopic arm assembly comprising a plurality of telescopically arranged arms and each arm mounting a receiving formation of the carrier.

According to a fourth aspect of the invention, there is provided a cradle for a catheter control assembly as described above, the cradle including
an elongate body member;
a plurality of relatively displaceable receiving formations arranged proximate a first end of the body member, each receiving formation being configured to receive a part of a catheter handle, the parts of the catheter handle being displaceable relative to one another; and
a mounting arrangement arranged at an opposed end of the body member for mounting the body member to a displacement mechanism of a catheter control assembly.

The cradle may be disposable and the mounting arrangement may be configured to mount the body member releasably on the displacement mechanism of the catheter control assembly.

The mounting arrangement may comprise a plurality of independently displaceable mounting members and each receiving formation may be connected to an associated mounting member via a pair of transversely spaced limbs. The limbs on each side of the body member may be shaped to nest within each other.

### Brief Description of Drawings

Fig. 1 shows a three dimensional view of an embodiment of a catheter control assembly;
Fig. 2 shows a three dimensional, front view of part of the assembly;
Fig. 3 shows a three dimensional, rear view of the part of the assembly of Fig. 2;
Fig. 4 shows a three dimensional view of a part of a displacement mechanism and a drive arrangement of the assembly;
Fig. 5 shows a three dimensional view of a further part of the assembly including the part of the displacement mechanism and the drive arrangement of Fig. 4;
Fig. 6a shows a three dimensional front view of a carrier of the assembly;
Fig. 6b shows a three dimensional rear view of the carrier of the assembly;
Fig. 7 shows a sectional end view of the carrier taken along line VII-VII in Fig. 6;
Fig. 8 shows a three dimensional view of an embodiment of a controller of the assembly;
Fig. 9 shows a side view of another embodiment of the controller of the assembly;
Fig. 10 shows a three dimensional rear view of a part of another embodiment of a catheter control assembly;
Fig. 11 shows a three dimensional front view of a part of a further embodiment of a catheter control assembly;
Fig. 12 shows a three dimensional rear view of a part of the further embodiment of the catheter control assembly of Fig, 11; and
Fig. 13 shows a three dimensional rear view, from an opposite side, of a part of the further embodiment of the catheter control assembly of Fig. 11.

### Detailed Description of Exemplary Embodiments

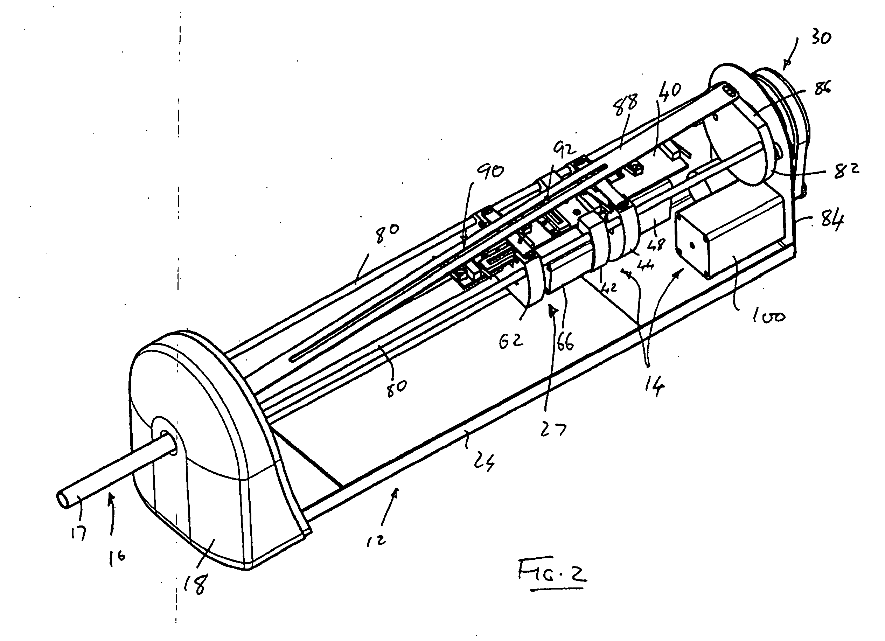

In the drawings, reference numeral 10 generally designates an embodiment of a catheter control assembly. The assembly 10 includes a support structure in the form of a housing 12. The housing 12 houses a driver arrangement 14 (Fig. 2). A displacement mechanism 16 is carried by the housing 18. The displacement mechanism 16 supports a carrier 20 on its free end. The carrier 20 is shaped to accommodate at least a part of a catheter handle 22 and in this embodiment is in the form of a cradle 21.

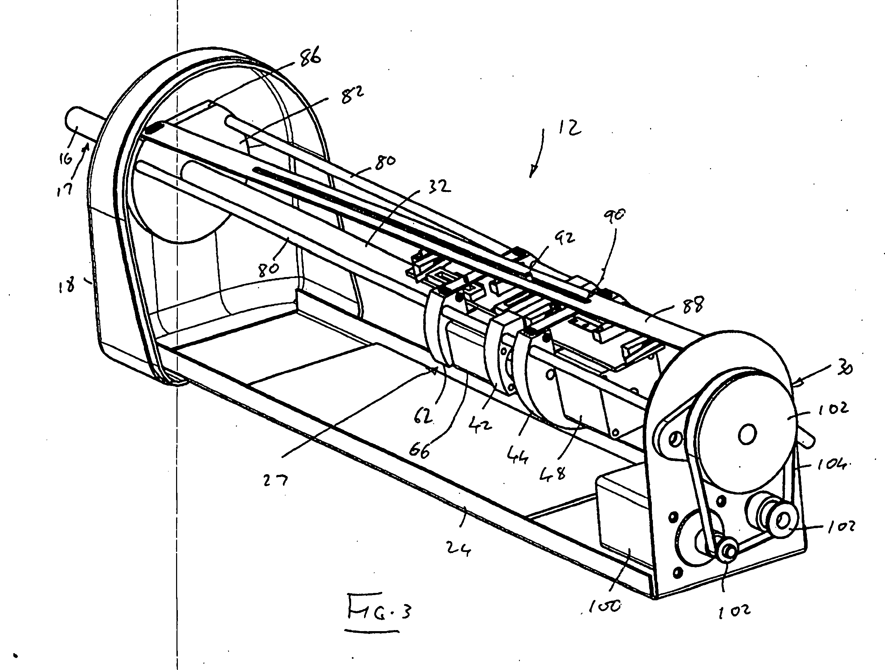

The housing 12 has a base 24 and a lid portion 26 removably mounted on the base 24. The lid portion 26 is removable to enable access to be gained to those parts of the displacement mechanism 16 and the drive arrangement 14 arranged within the housing 12 as shown in greater detail in Figs. 2 and 3 of the drawings. The parts of the displacement mechanism 16 and the drive arrangement 14 will be referred to collectively below as the internal assembly 27.

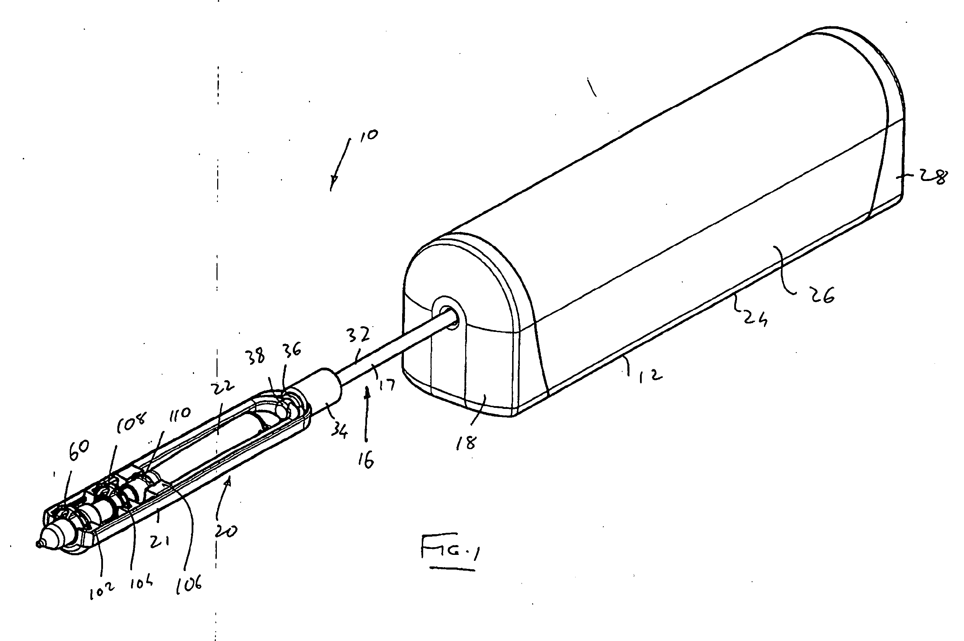

The housing 12 further includes a rear cover 28 (Fig. 1). The rear cover 28 is removable to expose a further part 30 of the displacement mechanism 16, as will be discussed in greater detail below.

The displacement mechanism 16 comprises a telescopic arm assembly 17 having a first, outer arm 32 carrying a mounting formation (not shown in this embodiment) at its free end on which a mounting member 34 of the cradle 21 is receivable. A second arm (also not shown in this embodiment) is nested within the arm 32 and mounts a second mounting member 36 of the cradle 21. A third arm (also not shown) of the telescopic arm assembly 17 is telescopically received within the second arm and carries a mounting formation for mounting a third mounting member 38 of the cradle 21. Thus, it will be appreciated that the arms are nested within each other and are telescopically arranged with respect to each other.

The internal assembly 27 includes a slide 40. A mounting plate 42 is fixedly mounted to the slide 40.

An electrode sheath of a catheter manufactured in accordance with the applicant's International Patent Application No. PCT/AU01/01339 dated 19 October 2001 and entitled "An electrical lead" has a lumen. The electrode sheath has an inner tubular member. Electrical conductors for electrodes at a distal end of the catheter are wound about the inner tubular member and the conductors are encased in a jacket. With this arrangement, the lumen is unimpeded and facilitates insertion of a steering shaft into the lumen for controlling steering and/or deflection of a distal end of the catheter. The steering shaft is made in accordance with the teachings of the applicant's International Patent Application No. PCT/AU2005/000216 dated 18 February 2005 and entitled "A steerable catheter". As described in that patent specification, the steering shaft has an outer, tubular member with a bend-enhancing region formed proximate a distal end of the tubular member. An inner actuator is received within the tubular member. Relative displacement between the actuator and the tubular member causes bending or deflection of the distal part of the steering shaft at the bend-enhancing region. As described in International Patent Application No. PCT/AU2005/000216, relative movement is achieved by attaching the tubular member of the steering shaft to a handle body of the catheter handle 22 with the actuator being attached to a mounting member 24 of the handle via a slide (not shown) within the handle 22. Relative movement between the slide and the handle body causes deflection at the distal end of the steering shaft.

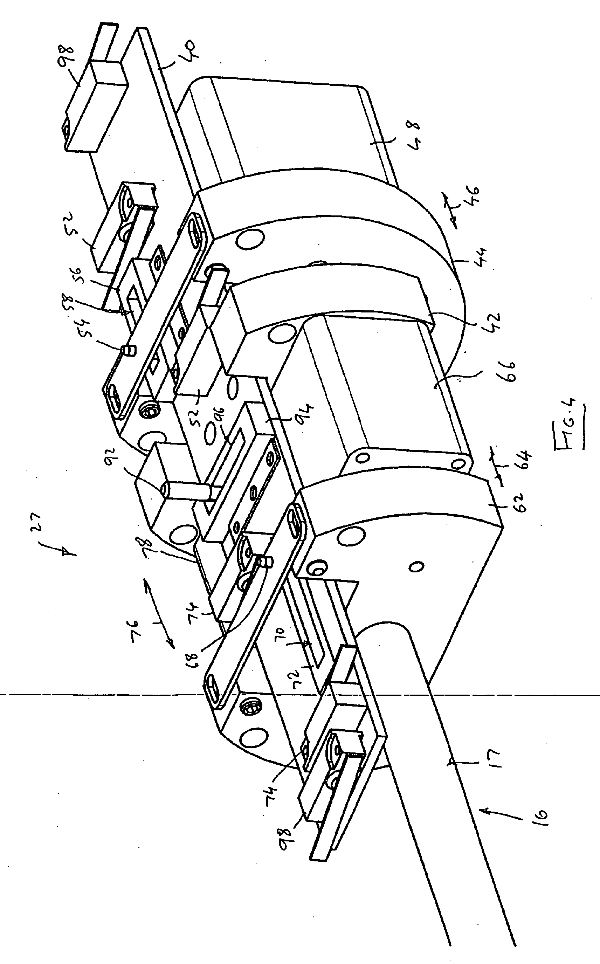

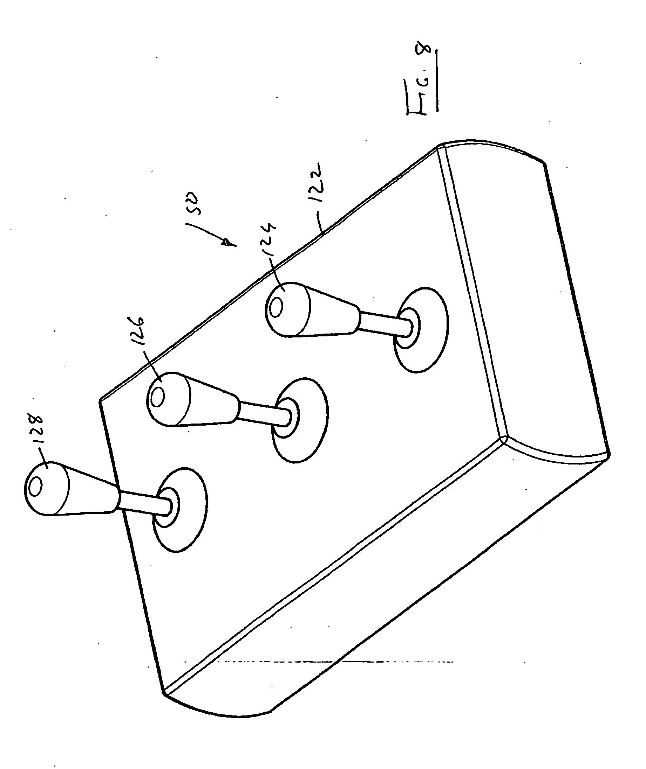

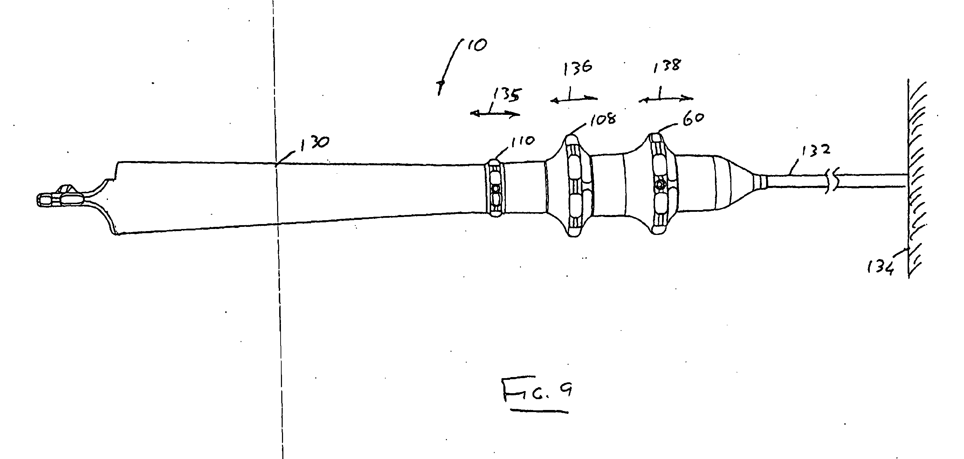

Thus, the internal assembly 27 further includes a second mounting plate 44 displaceably arranged relative to the mounting plate 42 in the direction of arrows 46 (Fig. 4). The innermost arm of the arm assembly 17 of the displacement mechanism 16 is fast with the mounting plate 44 while the middle arm of the arm assembly 17 of the displacement mechanism 16 is fast with the mounting plate 42. The mounting plate 44 mounts a drive motor 48 of the drive arrangement 14. The drive motor 48 is a stepper motor for effecting discrete, segmental motion of the mounting plate 44 relative to the mounting plate 42 under the action of a controller 50 (Figs. 8 and 9) of the assembly 10. The operation of the controllers 50 will be described in greater detail below.

It will be noted that the mounting plate 44 is displaceable relative to the mounting plate and the slide 40. The slide 40 supports limit switches 52 which limit the displacement of the mounting plate 44 relative to the mounting plate 42. A linear distance sensor 56, such as a linear potentiometer, has a pin 54 extending through a slot 58 of the sensor 56. The pin 54 is connected to the mounting plate 44 so that movement of the pin 54 as the plate 44 moves provides a displacement measurement.

Another benefit afforded by the modularity of the applicant's catheters is that the electrode sheath can be displaced independently of the steering shaft. This can be of benefit where one wishes to access awkward sites in a patient's body or to enhance tissue-electrode contact. The catheter handle 22 therefore includes a projection control knob 60 which can be used for extending and retracting the distal end of the electrode sheath relative to the steering shaft of the catheter.

The internal assembly 27 therefore includes a further mounting plate 62 which is arranged on an opposite side of the mounting plate 42 relative to the mounting plate 44. The mounting plate 62 supports the outermost arm 32 of the arm assembly 17 of the displacement mechanism 16.

The mounting plate 62 is displaceable in the direction of arrows 64 relative to the mounting plate 42 and the slide 40 under the action of a further drive motor 66 of the drive arrangement 14 of the assembly 10. A further linear distance sensor 72, which, once again, may be in the form of a linear potentiometer, is mounted on the slide 40 and is associated with the mounting plate 62. An actuation pin 68 is received through a slot 70 of the linear sensor 72. The pin 68 projects from the mounting plate 62 through the slot and provides a displacement measurement of the movement of the mounting plate 62 relative to the mounting plate 42. Limits of movement of the mounting plate 62 relative to the mounting plate 42 are controlled by a pair of limit switches 74 arranged at opposed ends of the linear sensor 72.

It is also desirable to extend and retract the entire telescopic arm assembly 17 relative to the housing 12. This is achieved by displacing the internal assembly 27 in the direction of arrows 76. A drive motor 78 (Fig. 4) of the drive arrangement 14 controls displacement of the internal assembly 27 in the direction of arrows 76. The drive motor 78 advances the mounting plate 42 along a pair of transversely spaced, longitudinally extending bars 80 (Figs. 2 and 3). The bars 80 are supported on a pair of opposed discs 82. One of the discs 82 is mounted on an inside surface of the end plate 18 with the other disc 82 being mounted on an inner surface of an opposed end wall 84 of the housing 12.

Each disc 82 has a flattened peripheral portion 86. A guide beam 88 is mounted fast with the flattened portions 86 of the discs 82. The guide beam 88 is arranged at an acute angle relative to a longitudinal axis of the housing 12. -The beam 88 defines a longitudinally extending slot 90. The slot 90 receives an actuator pin 92 of a further transversely arranged linear sensor 94 carried on the slide 40, the linear sensor 94 defining a slot 96 through which the pin 92 extends. The linear sensor 94 provides a displacement measurement of the movement of the telescopic arm assembly 17 relative to the housing 12. The angled arrangement of the guide beam 88 means that approximately 20 mm of movement of the pin 92 in the slot 96 of the linear sensor 94 translates into approximately 200 mm of motion in the direction of arrows 76. The limits of axial movement in the direction of arrows 76 is controlled by limit switches 98 mounted at opposed ends of the slide 40.

The discs 82 are rotatably supported in the housing 12. The drive arrangement 14 includes a drive motor 100 mounted on an inner surface of the end wall 84. The drive motor 100 rotatably drives the discs 82 via pulleys 101 and a drive belt 103.

Each of the drive motors 48, 66, 78 and 100 is a stepper motor for enabling incremental, discrete steps to be controlled by the controller 50 depending on the required movement.

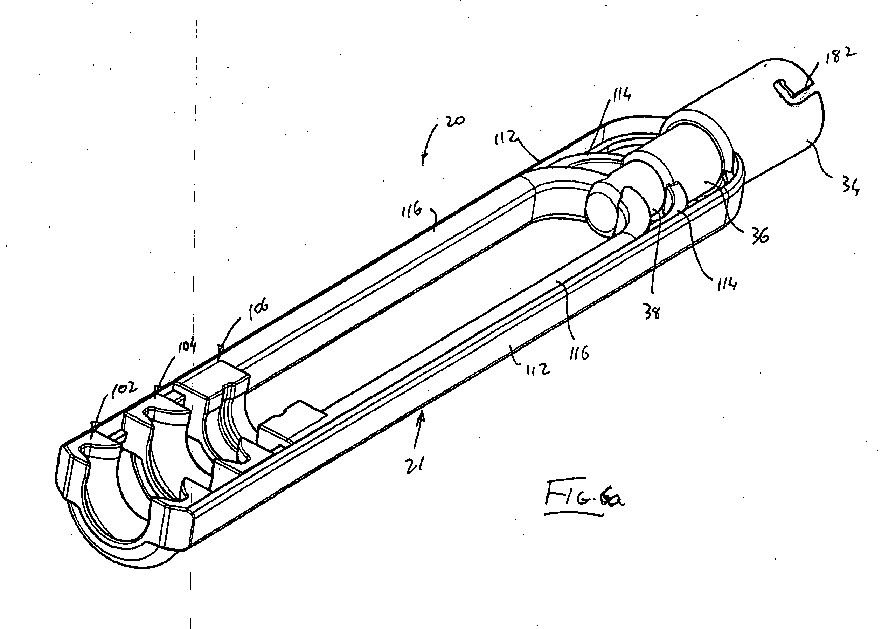

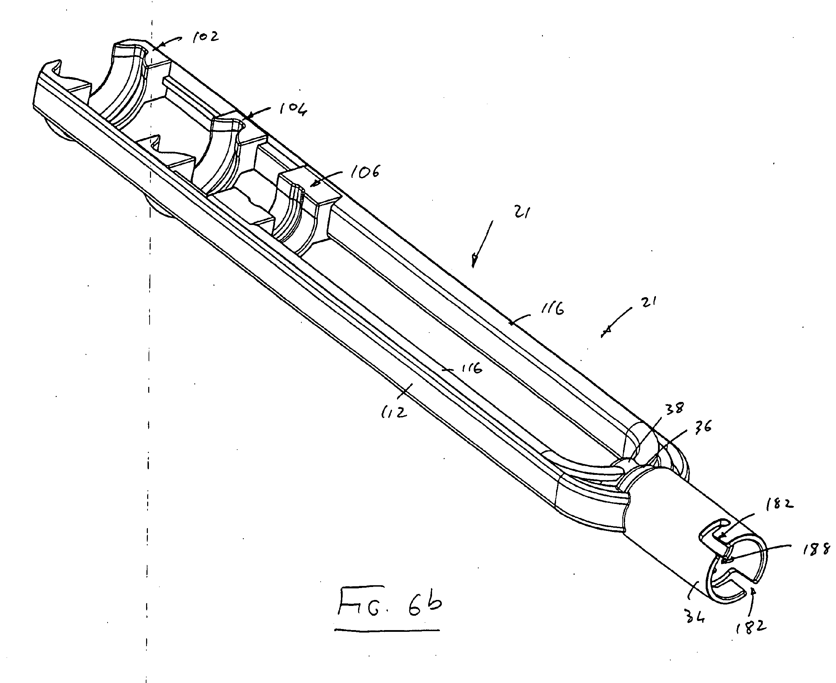

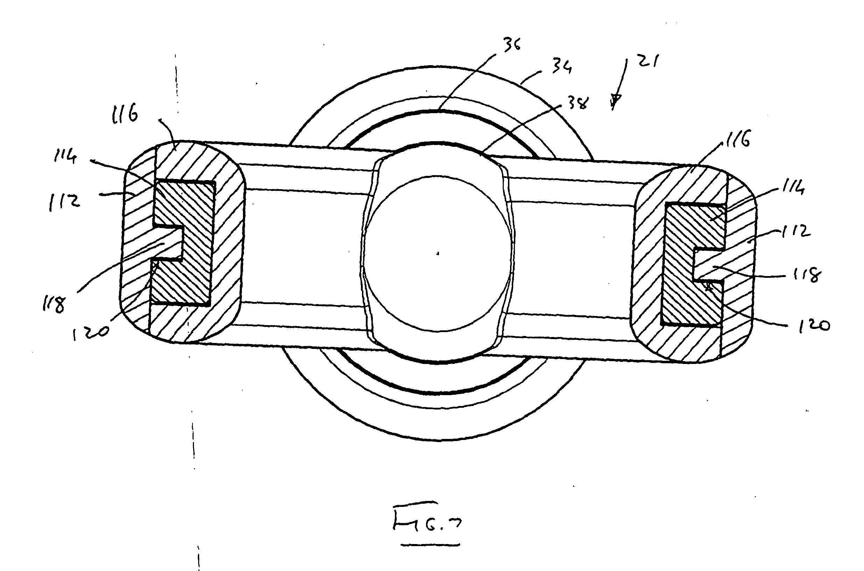

Referring now to Figs. 6a, 6b and 7 of the drawings, the cradle 21 is described in greater detail. In addition to the three, telescopically arranged mounting members 34, 36 and 38, the cradle 21 includes a plurality of receiving formations 102, 104 and 106. The receiving formation 102 receives the projection control knob 60 of the handle 22. The receiving formation 104 receives a steering control knob 108 of the handle 22 and the receiving formation 106 receives a circumferentially arranged raised, ridged abutment 110 arranged proximally of the steering control knob 108 and fast with the handle body of the catheter handle 22.

The receiving formation 102 is connected to the mounting member 34 by a pair of transversely spaced limbs 112. The receiving formation 104 is connected to the mounting member 36 by a pair of transversely spaced limbs 114 and, similarly, the receiving formation 106 is connected to the mounting member 38 by a pair of transversely spaced limbs 116. As shown more clearly in Fig. 7 of the drawings, each limb 114 nests within its associated limb 116 and is held captive between the limbs 112 and 116. Further, the limb 112 defines an inwardly projecting guide rib 118 which is received in a groove 120 of the limb 114 for facilitating guiding motion of the limbs 112, 114 and 116 relative to each other.

This nested arrangement of the limbs 112, 114 and 116 improves the stability of the cradle 21 and, more particularly, displacement of the receiving formations 102, 104 and 106 relative to one another. The nested arrangement of the limbs 112, 114 and 116 also serves to constrain the arms of telescopic arm assembly 17 against rotation relative to one another. It also creates a more compact cradle 21.

Referring to Figs. 8 and 9 of the drawings, two embodiments of a controller 50 of the assembly 10 are shown. In the case of the controller 50 shown in Fig. 8 of the drawings, the controller 50 includes a casing 122 from which three joysticks 124, 126 and 128 project. The controller 50 is either hardwired to the drive arrangement 14 of the assembly 10 or, instead, the controller 50 communicates wirelessly with the drive arrangement 14 of the assembly 10.

In either case, the joysticks 124, 126 and 128 are used for controlling manipulation of the catheter handle 22. More particularly, the joystick 124 performs two functions. The joystick 124 acts on the motor 100 so that moving the joystick 124 left and right rotates the internal assembly 27 counter-clockwise and clockwise, respectively. Moving the joystick 124 forward and backward acts on the motor 78 to cause forward and backward axial displacement of the internal assembly 27 in the direction of arrows 76, respectively.

The joystick 126 controls steering and/or deflection of the catheter. Thus, moving the joystick 126 forward and backwards control the motor 48 and effects axial displacement of the plate 44 with respect to the plate 42 in the direction of arrows 46. Thus, moving the joystick 126 forwards effects deflection of the catheter tip and moving the joystick 126 backwards cancels out the deflection.

The joystick 128 also performs a single function. The joystick 128 acts on the motor 66 and effects projection of the electrode sheath of the catheter relative to the steering shaft of the catheter. Thus, moving the joystick 128 forwards causes the catheter sheath to be projected from the steering shaft of the catheter and moving the joystick 128 backwards retracts the catheter sheath back on to the steering shaft.

In Fig. 9 of the drawings, another embodiment of a controller 50 is shown. In this embodiment, the controller 50 mimics the catheter handle 22. Thus, the controller 50 has a handle body 130 carrying a displaceable projection control knob 60, a displaceable steering control knob 108 and the raised, ridged abutment 110. The catheter body 130 is fast with a guide rod 132. The guide rod 132, in turn, is slidably, rotatably and pivotally mounted relative to a fixed support member 134.

With this arrangement, the clinician uses the handle controller 50 as though using the catheter handle 22 itself. Thus, to effect displacement of the entire catheter along the longitudinal axis of the catheter, the clinician 50 moves the handle controller 50 in the direction of arrows 135 on the guide rod 132. To effect steering or deflection of the catheter tip, the clinician uses the steering control knob 108 and moves it in the direction of arrows 136. To effect projection of the electrode sheath relative to the steering shaft, the clinician moves the projection control knob 60 in the direction of arrows 138 to act on the motor 66 to cause extension of the electrode sheath relative to the steering shaft or to retract the electrode sheath on to the steering shaft, as the case may be.

Similarly, to effect rotation of the entire catheter handle 22, the clinician rotates the controller 50 and the guide rod 132 relative to the fixed support member 134. The controller 50 is able to be rotated relative to the fixed support member 134 to improve wrist comfort for the clinician. Preferably, it is necessary to raise the controller 50 from its rest position (not shown) to its illustrated, operative position, by pivoting the rod 132 about a pivot point (not shown) before the actuation of any of the drive motors is enabled so that the assembly 10 cannot be inadvertently actuated.

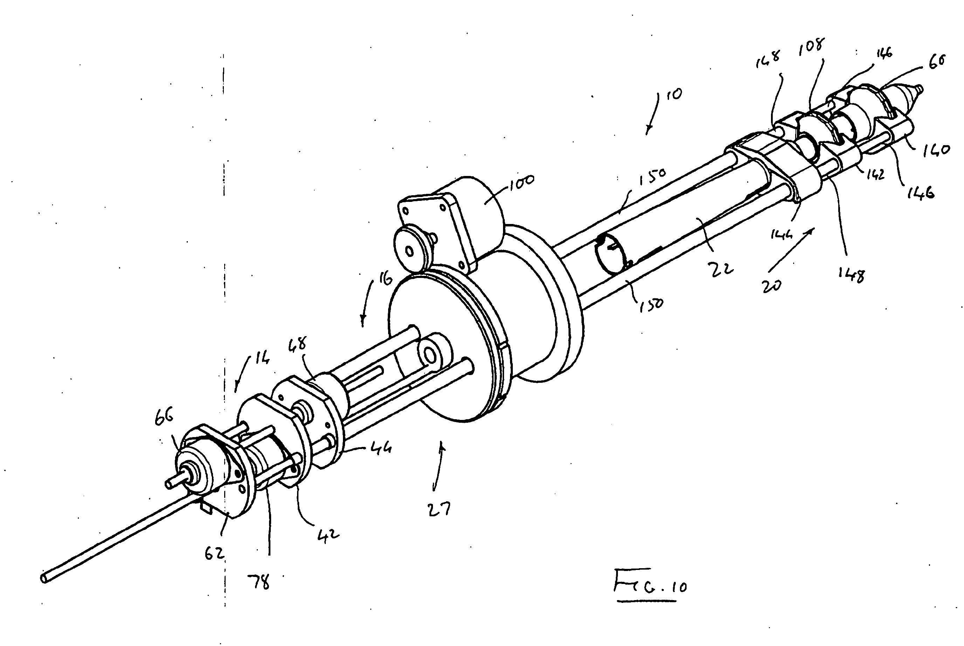

With reference to Fig. 10 of the drawings, another embodiment of the assembly 10 is illustrated. With reference to the previous drawings, like reference numerals refer to like parts, unless otherwise specified.

In this embodiment of the invention, rather than a dedicated cradle, the carrier 20 comprises three discrete components, each defining a receiving formation 140, 142 and 144. The projection control knob 60 is received in the receiving formation 140, the steering control knob 108 is received in the receiving formation 142 and the raised abutment 110 (not visible in Fig. 10) is received in the receiving formation 144.

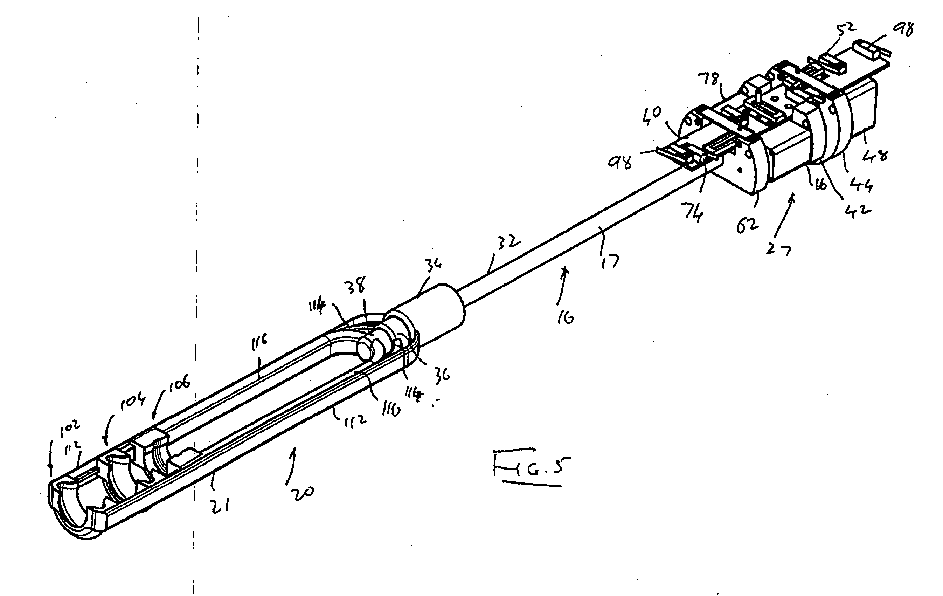

Also, instead of the displacement mechanism 16 comprising a telescopic arm assembly 17 where there are single nested arms, an arm comprising a pair of transversely spaced arm members 146, 148 and 150, is associated with each receiving formation 140, 142 and 144, respectively. Each arm member 146 is telescopically received in its associated arm member 148 which, in turn, is telescopically received in its associated arm member 150. The operation of this embodiment is similar to that described above with reference to Figs 1-5.

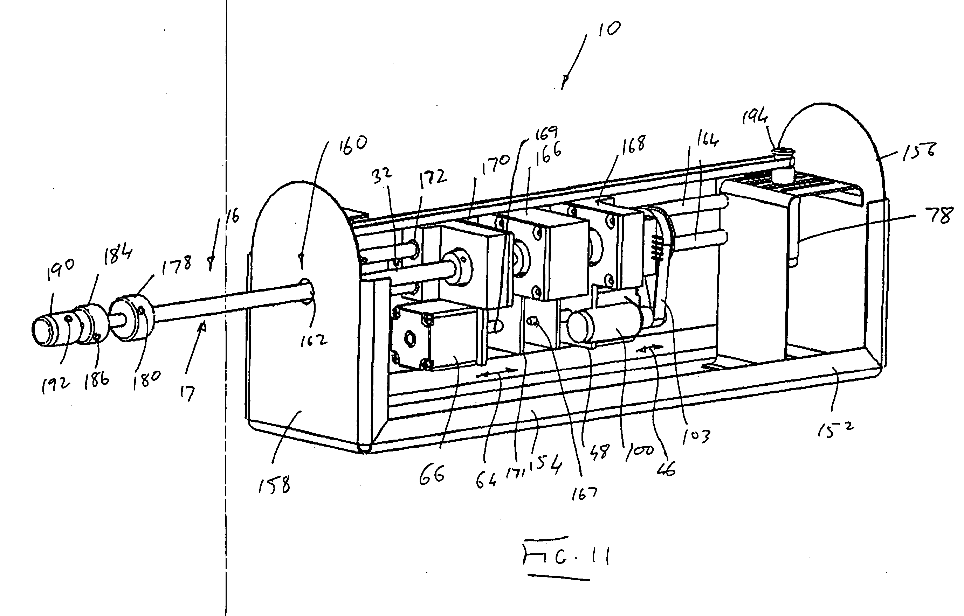

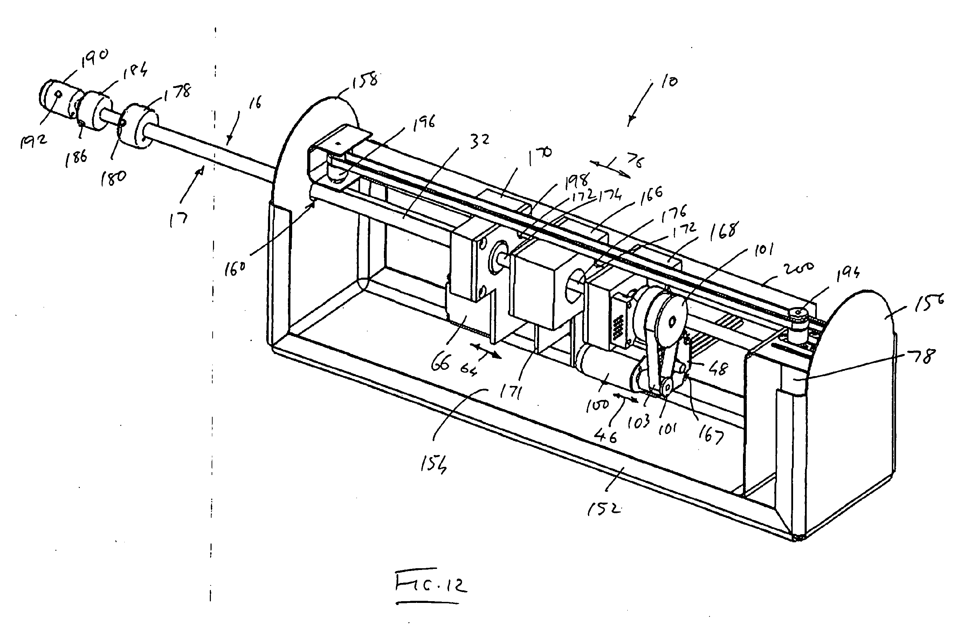

Referring now to Figs. 11 and 12 of the drawings, yet a further embodiment of a catheter control assembly 10 is illustrated. With reference to the previous drawings, like reference numerals refer to like parts, unless otherwise specified.

In this embodiment, the support structure includes a chassis 152 received in the housing 18. The housing 18 is omitted from Figs. 11 and 12 for the sake of clarity. The chassis 152 has a base member 154 and a pair of opposed end plates 156 and 158. The telescopic arm assembly 17 projects through an opening 160 in the end plate 158. A bearing 162 is received in the opening 162 to improve the stability of telescopic arm assembly 17.

The internal assembly 27 includes a pair of spaced, parallel guide rails 164 extending between the plates 156 and 158 of the chassis 152. A drive block assembly 166 is slidably supported on the guide rails 164 to be displaceable in the direction of arrows 76 to effect axial displacement of the cradle 21 (not shown in this embodiment) mounted on the end of the telescopic arm assembly 17 to effect axial displacement of the entire catheter, as described above with reference to Figs. 1-5 of the drawings.

A second drive block assembly 168 is displaceably arranged relative to the drive block assembly 166. The drive block assembly 168 is also mounted on the rails 164 and carries the drive motor 48. The drive motor 48 is mounted on a lead screw 167 which is fast with a bracket 169 suspended from the drive block assembly 166. Thus, rotational motion of the drive motor 48 translates into linear motion relative to the lead screw 167.

A third drive block assembly 170 is mounted on the guide rails 164 and supports the drive motor 66. The drive block assembly 168 is mounted on one side of the drive block assembly 166 with the drive block assembly 170 being mounted on an opposed side of the drive block assembly 166. The drive motor 66 is mounted on a lead screw 171 which is fast with the bracket 169 suspended from the drive block assembly 166 so that rotational motion of the drive motor 66 translates into linear motion relative to the lead screw 171.

Each of the drive block assemblies 166, 168 and 170 is mounted on the guide rails 164 via bearings 172 to facilitate displacement of the drive block assemblies 166, 168 and 172 on the guide rails 164.

In this embodiment, the telescopic arm assembly 17 is shown more clearly, particularly in Fig. 12 of the drawings. Thus, in addition to the outer arm 32, the telescopic arm assembly 17 includes a central arm 174 (Fig. 12) and the inner arm 176.

The outer arm 32 of the telescopic arm assembly 17 is mounted to the drive block assembly 170, the arm 174 is mounted to the drive block assembly 166 and the arm 176 is mounted to the drive block assembly 168.

The outer arm 32 of the telescopic arm assembly 17 is connected to the mounting member 34 (Figs. 6A and 6B) of the cradle 21 via a first mounting formation 178 of the displacement mechanism 16. The mounting formation 178 has a pair of opposed radially outwardly extending pins 180 (only one of which is shown). The pins 180 engage a pair of opposed L-shaped slots 182 (Figs. 6A and 6B) of the mounting member 34 of the cradle 21 bayonet fashion.

The central arm 174 supports a mounting formation 184 at its distal end. Once again, the mounting formation 184 has a pair of opposed radially outwardly extending pins 186. The pins 186 engage a pair of opposed L-shaped slots 188 (Figs. 6B) of the mounting member 36 of the cradle 21 bayonet fashion.

Finally, the inner arm 176 supports a mounting formation 190 at its distal end. The mounting formation 190 has a pair of opposed, radially outwardly extending pins 192. These pins 192 engage a pair of opposed L-shaped slots (not visible in the drawings) of the mounting member 38 of the cradle 21 bayonet fashion.

In this embodiment, axial displacement of the drive block assembly 166 is controlled by the stepper motor 78. The stepper motor 78 drives a drive pulley 194 (Figs. 11 and 12) arranged at a first end of the chassis 152. A driven pulley 196 is arranged at an opposed end of the chassis 152 from the pulley 194. A drive belt 198, to which the drive block assembly 166 is connected, extends about the pulleys 194 and 196. Thus, operation of the drive motor 78 effects axial displacement of the drive block assembly 166 in the direction of the arrows 76 to effect axial displacement of the entire cradle 21 and, hence, advancing and/or retracting the catheter, the handle 22 of which is received in the cradle 21, in use.

To effect rotation of the entire displacement mechanism 16, the stepper motor 100 is mounted on the drive block assembly 168 and rotates the inner arm 176 and, in so doing, the entire telescopic arm assembly 17 by means of the pulleys 101 and the drive belt 103. As described above, the nested limb arrangement of the cradle 21 constrains the arms 32, 174 and 176 of the telescopic arm assembly 17 against rotation relative to one another. Also, the arms can rotate relative to the drive block assemblies 166, 168 and 170 by means of bearings arranged in the drive block assemblies 166, 168 and 170.

To effect projection of a catheter sheath of the catheter relative to a steering shaft of the catheter sheath, the stepper motor 66 is operated by the clinician using the relevant controller 50. This displaces the drive block assembly 170 relative to the drive block assembly 166 in the direction of arrows 64 and, in turn, displaces the mounting member 34 of the cradle 21 relative to the other mounting members 36 and 38. As described above, the projection control knob 60 is received in the receiving formation 102 and displacement of the mounting member 34 relative to the other mounting members 36 and 38 displaces the receiving formation 102 relative to the other receiving formations 104 and 106 in an axial direction. This effects projection and retraction of the catheter sheath relative to the steering shaft of the catheter.

To effect steering and/or deflection of a distal end of the catheter, the stepper motor 48 is operated by the clinician using the controller 50. This effects displacement of the drive block assembly 168 in the direction of arrows 46 and, in turn, displacement of the mounting formation 190 of the displacement mechanism 16 relative to the-other mounting formations 178 and 184. Likewise, because the mounting member 38 of the cradle 21 is mounted on the mounting formation 190, displacement of the drive block assembly 168 similarly effects displacement of the mounting member 38 of the cradle 21 relative to the other mounting members 34 and 36.

When the stepper motor 48 is operated to effect steering and/or deflection, the mounting member 38 is displaced axially relative to the other mounting members 34 and 36. This causes the receiving formation 106, containing the raised, ridged abutment 110 of the catheter handle 22 to be displaced relative to the steering control knob 108 received in the receiving formation 104. This effects steering and/or deflection of the distal end of the catheter.

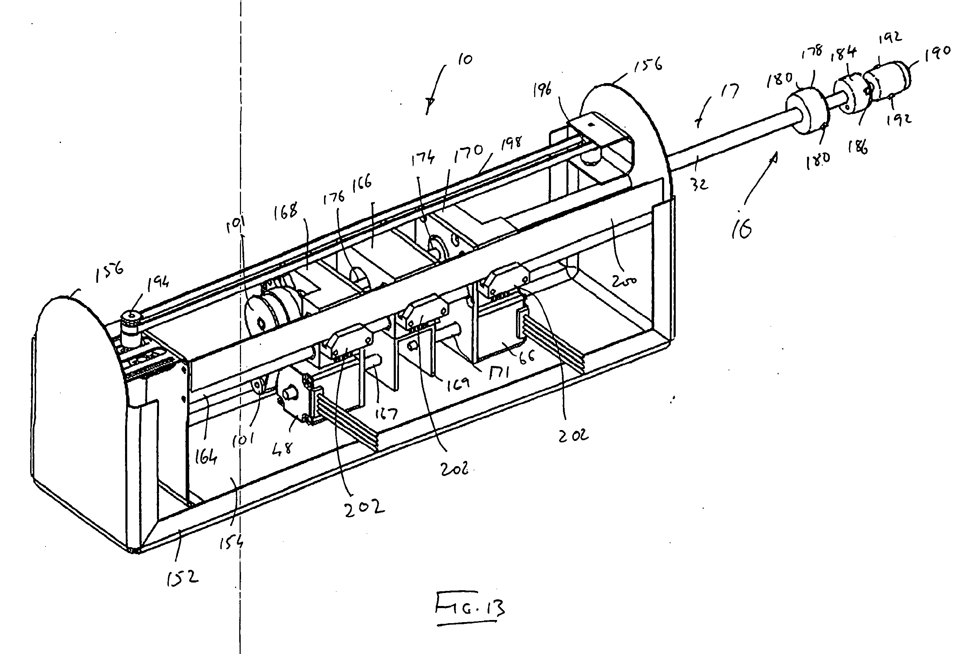

It is to be noted that, in this embodiment, linear potentiometers and limit switches can also be used to control and limit motion of the displacement mechanism 16. Instead, as illustrated in Fig. 13 of the drawings, optical encoders and optical interrupters are used. Thus, a length of optical tape 200 extends alongside the guide rails 164 between the end plates 156, 158 of the chassis 152. Each drive block assembly 166, 168 and 170 carries an incremental optical encoder 202 for enabling absolute positioning of the drive block assemblies 166, 168 and 170 to be determined.

It is an advantage of the invention that a catheter control assembly 10 is provided which facilitates stable control of a catheter and obviates problems arising from vibrations, etc. In addition, the catheter control assembly 10 is able to be operated remotely by a clinician thereby taking the clinician out of a field of radiation in which the clinician may otherwise need to operate. Greater stability of the catheter using the control assembly 10 also improves accuracy of procedures carried out by the clinician. This stability is enhanced by the use of the telescoped arms which, in addition, reduces the size and footprint of the assembly 10.

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. A catheter control assembly (10) which includes
a support structure (12);
a drive arrangement (14) supported by the support structure (12);
an elongate displacement mechanism (16) associated with the drive arrangement (14) to be displaced at least axially under the action of the drive arrangement (14); and
a carrier (20), for mounting at least a part of a catheter handle (22), mounted on the displacement mechanism (16), the carrier (20) comprising a plurality of independently displaceable receiving formations (102, 104, 106), each receiving formation (102, 104, 106), in use, receiving a part of the catheter handle (22) and at least two of the receiving formations (102, 104, 106) being axially displaceable relative to each other **characterised in that** the displacement mechanism (16) includes a telescopic arm assembly (17) extending from the support structure (12) and on which the carrier (20) is mounted, the telescopic arm assembly (17) comprising a plurality of telescopically arranged arms (32) and each arm mounting one of the receiving formations (102, 104, 106) of the carrier.

2. The assembly (10) of claim 1 in which the telescopic arm assembly (17) is at least axially and rotatably arranged relative to the support structure (12) for effecting axial and rotational displacement of the carrier (20) relative to the support structure (12), the telescopic arm assembly (17) further including at least a first arm (32) and a second arm, the second arm of the telescopic arm (17) assembly displacing one receiving formation (102, 104, 106) of the carrier (20) relative to at least one other receiving formation (102, 104, 106) of the carrier (20).

3. The assembly (10) of claim 2 in which the telescopic arm assembly (17) includes at least one further arm for displacing another receiving formation (102, 104, 106) of the carrier (20) relative to at least one other receiving formation (102, 104, 106) of the carrier (20).

4. The assembly (10) of any one of the preceding claims in which the carrier (20) is removably attached to the telescopic arm assembly (17).

5. The assembly (10) of any one of the preceding claims which includes a controller (50) for controlling operation of the drive arrangement (14) to effect displacement of the displacement mechanism (16).

6. The assembly (10) of claim 5 in which the controller (50) is arranged remotely of the support structure (12).

7. The assembly (10) of claim 5 or claim 6 in which the controller (50) is shaped to mimic the appearance and feel of the catheter handle (22).

8. The assembly (10) of any one of the preceding claims in which the drive arrangement (14) comprises a plurality of drive motors (48, 66, 78), each drive motor (48, 66, 78) driving a part of the displacement mechanism (16), the drive arrangement (14) further including a rotation control motor (100) for effecting rotary control of the displacement mechanism (16).

9. The assembly (10) of any one of the preceding claims in which the carrier (20) is in the form of a cradle (21) in which at least a part of the catheter handle (22) is receivable.

10. The assembly (10) of claim 9 in which the cradle (21) includes
an elongate body member containing the receiving formations (102, 104, 106); and
a mounting arrangement (34, 36, 38) arranged at an opposed end of the body member for mounting the body member to the displacement mechanism (16).

11. The assembly (10) of claim 10 in which the mounting arrangement (34, 36, 38) comprises a plurality of independently displaceable mounting members (34, 36, 38) and in which each receiving formation (102, 104, 106) is connected to an associated mounting member (34, 36, 38) via a pair of transversely spaced limbs (112, 114, 116).

12. The assembly (10) of claim 11 in which the limbs (112, 114, 116) on each side of the body member are shaped to nest within each other.

13. The assembly (10) of any one of claim 1 to 8 in which the carrier (20) comprises a plurality of discrete components , each mounted on one of the arms of the telescopic arm assembly, each component defining a mounting formation (140, 142, 144) for receiving a part of the catheter handle (22).

14. A cradle (21) for a catheter control assembly (10) as claimed in claim 1, the cradle (21) including
an elongate body member;
a plurality of relatively displaceable receiving formations (102, 104, 106) arranged proximate a first end of the body member, each receiving formation (102, 104, 106) being configured to receive a part of a catheter handle (22), the parts of the catheter handle being displaceable relative to one another; and
a mounting arrangement (34, 36, 38) arranged at an opposed end of the body member for mounting the body member to a displacement mechanism (16) of the catheter control assembly (10).

15. The cradle (21) of claim 14 which is disposable and in which the mounting arrangement (34, 36, 38) is configured to mount the body member releasably on the displacement mechanism (16) of the catheter control assembly (10).

16. The cradle (21) of claim 14 or claim 15 in which the mounting arrangement (34, 36, 38) comprises a plurality of independently displaceable mounting members (34, 36, 38) and in which each receiving formation (102, 104, 106) is connected to an associated mounting member (34, 36, 38) via a pair of transversely spaced limbs (112, 114, 116).

17. The cradle of claim 16 in which the limbs (112, 114, 116) on each side of the body member are shaped to nest within each other.

## Patentansprüche

1. Kathetersteuerungsbaugruppe (10), die Folgendes umfasst:
eine Tragkonstruktion (12);
eine von der Tragkonstruktion (12) getragene Antriebsanordnung (14);
einen länglichen Verschiebungsmechanismus (16), der so mit der Antriebsanordnung (14) assoziiert ist, dass er unter der Wirkung der Antriebsanordnung (14) wenigstens axial verschoben wird; und
einen Träger (20) zum Befestigen von wenigstens einem Teil eines Kathetergriffs (22), der am Verschiebungsmechanismus (16) befestigt ist, wobei der Träger (20) mehrere unabhängig verschiebbare Aufnahmestrukturen (102, 104, 106) umfasst, wobei jede Aufnahmestruktur (102, 104, 106) im Gebrauch einen Teil des Kathetergriffs (22) aufnimmt und wenigstens zwei der Aufnahmestrukturen (102, 104, 106) relativ zueinander axial verschiebbar sind, **dadurch gekennzeichnet, dass** der Verschiebungsmechanismus (16) eine Teleskoparmbaugruppe (17) beinhaltet, die sich von der Tragkonstruktion (12) erstreckt und auf der der Träger (20) befestigt ist, wobei die Teleskoparmbaugruppe (17) mehrere teleskopisch angeordnete Arme (32) umfasst, wobei an jedem Arm eine der Aufnahmestrukturen (102, 104, 106) des Trägers befestigt ist.

2. Baugruppe (10) nach Anspruch 1, wobei die Teleskoparmbaugruppe (17) wenigstens axial und drehbar relativ zur Tragkonstruktion (12) angeordnet ist, um eine Axial- und Rotationsverschiebung des Trägers (20) relativ zur Tragkonstruktion (12) zu bewirken, wobei die Teleskoparmbaugruppe (17) ferner wenigstens einen ersten Arm (32) und einen zweiten Arm umfasst, wobei der zweite Arm der Teleskoparmbaugruppe (17) eine Aufnahmestruktur (102, 104, 106) des Trägers (20) relativ zu wenigstens einer anderen Aufnahmestruktur (102, 104, 106) des Trägers (20) verschiebt.

3. Baugruppe (10) nach Anspruch 2, wobei die Teleskoparmbaugruppe (17) wenigstens einen weiteren Arm zum Verschieben einer weiteren Aufnahmestruktur (102, 104, 106) des Trägers (20) relativ zu wenigstens einer anderen Aufnahmestruktur (102, 104, 106) des Trägers (20) umfasst.

4. Baugruppe (10) nach einem der vorherigen Ansprüche, wobei der Träger (20) an der Teleskoparmbaugruppe (17) entfernbar angebracht ist.

5. Baugruppe (10) nach einem der vorherigen Ansprüche, die ein Steuergerät (50) zum Steuern des Betriebs der Antriebsanordnung (14) umfasst, um die Verschiebung des Verschiebungsmechanismus (16) zu bewirken.

6. Baugruppe (10) nach Anspruch 5, wobei das Steuergerät (50) fern von der Tragkonstruktion (12) angeordnet ist.

7. Baugruppe (10) nach Anspruch 5 oder Anspruch 6, wobei das Steuergerät (50) so gestaltet ist, dass es Optik und Haptik des Kathetergriffs (22) nachahmt.

8. Baugruppe (10) nach einem der vorherigen Ansprüche, wobei die Antriebsanordnung (14) mehrere Antriebsmotoren (48, 66, 78) umfasst, wobei jeder Antriebsmotor (48, 66, 78) einen Teil des Verschiebungsmechanismus (16) antreibt, wobei die Antriebsanordnung (14) ferner einen Rotationssteuerungsmotor (100) zum Bewirken einer Rotationssteuerung des Verschiebungsmechanismus (16) umfasst.

9. Baugruppe (10) nach einem der vorherigen Ansprüche, wobei der Träger (20) in Form einer Aufnahmevorrichtung (21) vorliegt, in der wenigstens ein Teil des Kathetergriffs (22) aufnehmbar ist.

10. Baugruppe (10) nach Anspruch 9, wobei die Aufnahmevorrichtung (21) Folgendes umfasst:
ein längliches Körperelement, das die Aufnahmestrukturen (102, 104, 106) beinhaltet; und
eine Befestigungsanordnung (34, 36, 38), die an einem gegenüberliegenden Ende des Körperelements angeordnet ist, um das Körperelement am Verschiebungsmechanismus (16) zu befestigen.

11. Baugruppe (10) nach Anspruch 10, wobei die Befestigungsanordnung (34, 36, 38) mehrere unabhängig verschiebbare Befestigungselemente (34, 36, 38) umfasst und wobei jede Aufnahmestruktur (102, 104, 106) über ein Paar in Querrichtung beabstandete Glieder (112, 114, 116) mit einem assoziierten Befestigungselement (34, 36, 38) verbunden ist.

12. Baugruppe (10) nach Anspruch 11, wobei die Glieder (112, 114, 116) an jeder Seite des Körperelements so gestaltet sind, dass sie ineinander verschachtelt sind.

13. Baugruppe (10) nach einem der Ansprüche 1 bis 8, wobei der Träger (20) mehrere getrennte Komponenten umfasst, die jeweils auf einem der Arme der Teleskoparmbaugruppe befestigt sind, wobei jede Komponente eine Befestigungsstruktur (140, 142, 144) zum Aufnahmen eines Teils des Kathetergriffs (22) definiert.

14. Aufnahmevorrichtung (21) für eine Kathetersteuerungsbaugruppe (10) nach Anspruch 1, wobei die Aufnahmevorrichtung (21) Folgendes beinhaltet:
ein längliches Körperelement;
mehrere relativ verschiebbare Aufnahmestrukturen (102, 104, 106), die in der Nähe eines ersten Endes des Körperelements angeordnet sind, wobei jede Aufnahmestruktur (102, 104, 106) so konfiguriert ist, dass sie einen Teil eines Kathetergriffs (22) aufnimmt, wobei die Teile des Kathetergriffs relativ zueinander verschiebbar sind; und
eine Befestigungsanordnung (34, 36, 38), die an einem gegenüberliegenden Ende des Körperelements angeordnet ist, um das Körperelement an einem Verschiebungsmechanismus (16) der Kathetersteuerungsbaugruppe (10) zu befestigen.

15. Aufnahmevorrichtung (21) nach Anspruch 14, die wegwerfbar ist und bei der die Befestigungsanordnung (34, 36, 38) so konfiguriert ist, dass das Körperelement lösbar am Verschiebungsmechanismus (16) der Kathetersteuerungsbaugruppe (10) befestigt wird.

16. Aufnahmevorrichtung (21) nach Anspruch 14 oder Anspruch 15, wobei die Befestigungsanordnung (34, 36, 38) mehrere unabhängig verschiebbare Befestigungselemente (34, 36, 38) umfasst und wobei jede Aufnahmestruktur (102, 104, 106) mit einem assoziierten Befestigungselement (34, 36, 38) über ein Paar in Querrichtung beabstandete Glieder (112, 114, 116) verbunden ist.

17. Aufnahmevorrichtung nach Anspruch 16, wobei die Glieder (112, 114, 116) auf jeder Seite des Körperelements so gestaltet sind, dass sie ineinander verschachtelt sind.

## Revendications

1. Ensemble de commande de cathéter (10), incluant :
une structure support (12) ;
un agencement d'entraînement (14), supporté par la structure support (12) ;
un mécanisme de déplacement (16) allongé, associé à l'agencement d'entraînement (14), de manière à être déplacé au moins axialement sous l'action de l'agencement d'entraînement (14) ; et
un support (20), pour monter au moins une partie d'une poignée de cathéter (22), montée sur le mécanisme de déplacement (16), le support (20) comprenant une pluralité de formations réceptrices (102, 104, 106) déplaçables indépendamment, chaque formation réceptrice (102, 104, 106), en utilisation, recevant une partie de la poignée de cathéter (22), et au moins deux des formations réceptrices (102, 104, 106) étant déplaçables axialement par rapport à chaque autre, **caractérisé en ce que** le mécanisme de déplacement (16) comprend un ensemble à bras télescopique (17), s'étendant à partir de la structure support (12) et sur lequel le support (20) est monté, l'ensemble à bras télescopique (17) comprenant une pluralité de bras agencés de manière télescopique (32) et chaque bras servant au montage d'une des formations réceptrices (102, 104, 106) du support.

2. Ensemble (10) selon la revendication 1, dans lequel l'ensemble à bras télescopique (17) est agencé de manière à effectuer un déplacement au moins axial et en rotation par rapport à la structure support (12), pour effectuer un déplacement axial et en rotation du support (20) par rapport à la structure support (12), l'ensemble à bras télescopique (17) comprenant en outre au moins un premier bras (32) et un deuxième bras, le deuxième bras de l'ensemble à bras télescopique (17) déplaçant une formation réceptrice (102, 104, 106) du support (20) par rapport à au moins une autre formation réceptrice (102, 104, 106) du support (20).

3. Ensemble (10) selon la revendication 2, dans lequel l'ensemble à bras télescopique (17) comprend au moins un autre bras pour déplacer une autre formation réceptrice (102, 104, 106) du support (20) par rapport à au moins une autre formation réceptrice (102, 104, 106) du support (20).

4. Ensemble (10) selon l'une quelconque des revendications précédentes, dans lequel le support (20) est fixé de manière désolidarisable à l'ensemble à bras télescopique (17).

5. Ensemble (10) selon l'une quelconque des revendications précédentes, comprenant un contrôleur (50) pour commander le fonctionnement de l'agencement d'entraînement (14), de manière à effectuer un déplacement du mécanisme de déplacement (16).

6. Ensemble (10) selon la revendication 5, dans lequel le contrôleur (50) est disposé à distance de la structure support (12).

7. Ensemble (10) selon la revendication 5 ou la revendication 6, dans lequel le contrôleur (50) est conformé pour reproduire l'aspect et la sensation de la poignée de cathéter (22).

8. Ensemble (10) selon l'une quelconque des revendications précédentes, dans lequel l'agencement d'entraînement (14) comprend une pluralité de moteurs d'entraînement (48, 66, 78), chaque moteur d'entraînement (48, 66, 78) entraînant une partie du mécanisme de déplacement (16), l'agencement d'entraînement (14) comprenant en outre un moteur de commande de rotation (100) pour effectuer une commande de rotation du mécanisme déplacement (16).

9. Ensemble (10) selon l'une quelconque des revendications précédentes, dans lequel le support (20) se présente sous la forme d'un berceau (21) dans lequel au moins une partie de la poignée de cathéter (22) est susceptible d'être logée.

10. Ensemble (10) selon la revendication 9, dans lequel le berceau (21) comprend :
un organe formant corps allongé, contenant les formations réceptrices (102, 104, 106) ; et
un agencement de montage (34, 36, 38), agencé à une extrémité opposée de l'organe formant corps, pour monter l'organe formant corps sur le mécanisme de déplacement (16).

11. Ensemble (10) selon la revendication 10, dans lequel l'agencement de montage (34, 36, 38) comprend une pluralité d'organes de montage (34, 36, 38) déplaçables indépendamment, et dans lequel chaque formation réceptrice (102, 104, 106) est connectée à un organe de montage (34, 36, 38) associé, via une paire de branches (112, 114, 116) espacées transversalement.

12. Ensemble (10) selon la revendication 11, dans lequel les branches (112, 114, 116) situées sur chaque côté de l'organe formant corps sont conformées de manière à s'emboîter les unes dans les autres.

13. Ensemble (10) selon l'une quelconque des revendications 1 à 8, dans lequel le support (20) comprend une pluralité de composants discrets, chacun monté sur l'un des bras de l'ensemble à bras télescopique, chaque composant définissant une formation de montage (140, 142, 144) pour recevoir une partie de la poignée de cathéter (22).

14. Berceau (21) pour un ensemble de commande de cathéter (10) selon la revendication 1, le berceau (21) comprenant :
un organe formant corps allongé ;
une pluralité de formations réceptrices (102, 104, 106) déplaçables les unes par rapport aux autres, agencées à proximité d'une première extrémité de l'organe formant corps, chaque formation réceptrice (102, 104, 106) étant configurée pour recevoir une partie d'une poignée de cathéter (22), les parties de la poignée de cathéter étant déplaçables les unes par rapport aux autres ; et
un agencement de montage (34, 36, 38), agencé à une extrémité opposée de l'organe formant corps, pour monter l'organe formant corps sur le mécanisme de déplacement (16) de l'ensemble de commande de cathéter (10).

15. Berceau (21) selon la revendication 14, jetable et dans lequel l'agencement de montage (34, 36, 38) est configuré pour monter l'organe formant corps de manière désolidarisable sur le mécanisme de déplacement (16) de l'ensemble de commande de cathéter (10).

16. Berceau (21) selon la revendication 14 ou la revendication 15, dans lequel l'agencement de montage (34, 36, 38) comprend une pluralité d'organes de montage (34, 36, 38) déplaçables indépendamment, et dans lequel chaque formation réceptrice (102, 104, 106) est connectée à un organe de montage (34, 36, 38) associé, via une paire de branches (112, 114, 116) espacées transversalement.

17. Berceau selon la revendication 16, dans lequel les branches (112, 114, 116) situées sur chaque côté de l'organe formant corps sont conformées de manière à s'emboîter les unes dans les autres.
